# EUROPEAN PATENT SPECIFICATION

(11) **EP 1 844 008 B1**
(45) Date of publication and mention of the grant of the patent: **04.08.2010**
(21) Application number: 06706426.1
(22) Date of filing: 26.01.2006
(51) Int. Cl.: C07C 323/52, A61K 31/22

(54) **VALNEMULIN CRYSTALLINE SALTS WITH ORGANIC ACIDS**
KRISTALLINE VALNEMULINSALZE MIT ORGANISCHEN SÄUREN
SELS CRISTALLINS DE VALNEMULINE CONTENANT DES ACIDES ORGANIQUES

(30) Priority: 26.01.2005 EP 05001498
(43) Date of publication of application: 17.10.2007
(73) Proprietor: Novartis AG, 4056 Basel (CH)
(72) Inventor: MACHER, Ingolf, A-6300 Wörgl (AT); GEISSLER, Annett, 79576 Weil am Rhein-Haltingen (DE); WIELAND-BERGHAUSEN, Susanne, Christine, 79541 Lörrach (DE); SCHOTE, Uwe, Thomas, CH-4055 Basel (CH); RAKOCZI, Ferenc, Jozsef, CH-4132 Muttenz (CH)
(74) Representative: Liphardt, Bernd
(86) International application number: PCT/EP2006/000680
(87) International publication number: WO 2006/079535

(56) References cited:
- EP-A- 0 153 277
- EP-A- 0 524 632
- WO-A-98/01127
- WO-A-03/082260
- GOULD P L: "SALT SELECTION FOR BASIC DRUGS" 1986, INTERNATIONAL JOURNAL OF PHARMACEUTICS, AMSTERDAM, NL, PAGE(S) 201-217 , XP002074725 ISSN: 0378-5173 the whole document
- BERGE S M ET AL: "PHARMACEUTICALS SALTS" JOURNAL OF PHARMACEUTICAL SCIENCES, AMERICAN PHARMACEUTICAL ASSOCIATION. WASHINGTON, US, vol. 66, no. 1, 1977, pages 1-19, XP000562636 ISSN: 0022-3549

## Description

The present invention relates to the preparation of a new salt form of valnemulin, which is notable for its good crystallinity in higher purity, its simpler technical usage and improved storage stability both as the pure active ingredient and also used in formulations.

Valnemulin, a compound of formula I, is known from EP 0153277, and as a formulated product it is marketed under the trade name Econor^{®}.

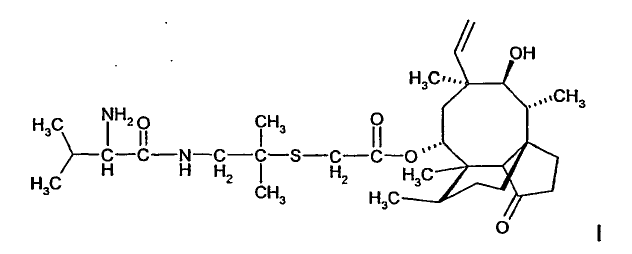

As is generally known, this compound has anti-bacterial properties upon e.g. oral or parenteral administration and is therefore used for the prevention or cure of a series of bacterial infections in the field of animal health. The broad spectrum of activity includes *e.g.Streptococcus aronson, Staphylococcus aureus, Mycoplasma arthritidis, Mycoplasma bovigenitalium, Mycoplasma bovimastitidis, Mycoplasma bovirhinis, Mycoplasma sp., Mycoplasma canis, Mycoplasma felis, Mycoplasma fermentans, Mycoplasma gallinarum, Mycoplasma gallisepticum, A. granularum, Mycoplasma hominis, Mycoplasma hyorhinis, Actinobacillus laidlawii, Mycoplasma meleagridis, Mycoplasma neurolyticum, Mycoplasma pneumonia* und *Mycoplasma hyopneumoniae.*

WO 98/01127 additionally describes the outstanding activity of these compounds against a disease complex which occurs under conditions in which the animals have to be kept in crowded conditions, e.g. for the purpose of transport, and are therefore exposed to great stress. The most frequent pathogens that play a decisive role under these conditions are *Mycoplasma hyopneumoniae, Brachyspira* (formerly *Serpulina* or *Treponema*) *hyodysenteriae, Brachyspira pilosicoli, Lawsonia intracellularis, Mycoplasma gallisepticum, Pasteurella multocida, Actinobacillus (Haemophilus) pleuromoniae* and *Haemophilus parasuis,* whereby diseases of the respiratory tract and other infections often occur together and lead to a complex clinical picture. Both herd animals and domestic animals are affected, e.g. cattle, sheep and pigs, chickens, dogs and cats.

Valnemutin as a free base is relatively unstable in storage and was therefore stabilised either in the form of the valnemulin-cyclodextrin complex (EP-0,524,632 or by microspherules (WO 03/45354), or used in the form of the free base prepared *in situ* (WO 01/41758) or predominantly in the form of the amorphous hydrochloride (EP-0,153,277, WO 98/01127, WO 01/37828). The only salt form described until now has been the amorphous hydrochloride, which is stable in storage as the pure substance and in the formulated product (Econor^{®}). However, this only applies to a very limited extent, as will be shown below, in a mixture with other substances, especially with feed.

As is known *inter alia* from European patent specification EP0524632, antibiotics from the group of pleuromutilins, which includes valnemulin named herein, may be added to drinking water very simply when in the form of the water-soluble hydrochloride. In contrast, however, it has proved difficult to administer these antibiotics to the animals requiring treatment via the feed, since these antibiotics are broken down very rapidly by the feed components and are thus inactivated. However, when producing mixtures of feed and medicament, it is essential that a certain degree of stability in storage can be attained, as otherwise it is impossible to provide precise dosaging. Therefore, even earlier, various efforts were made to improve the stability of valnemulin in meal feed and in pelleted feed.

Whereas antibiotics can be administered to humans in the most varied application forms, such as tablets, coated tablets, emulsions, injection solutions and the like, because one can rely on the discipline and desire to recover in human patients, in the case of animals considerable practical problems are encountered.

An animal must have a natural willingness to take a medicinal preparation orally. Of course, an individual animal or a few animals can also be forced to take an antibiotic, by making it swallow or by injecting it. However, these methods using force are unacceptable to large animal holdings, as they are labour-intensive, require the veterinarian in each individual case and therefore lead to high costs. Therefore, in the management of groups of animals, simple and safe application forms must be found, especially those which are considerate to the animals, and which are either taken willingly by the animal or if enforced treatment is necessary, can be administered by the veterinarian, or where permitted, by the animal keeper himself or even fully automatically, and which keep costs within a tolerable limit.

One method which takes these circumstances into consideration is the correctly dosed administration of antibiotics incorporated into dry animal feed, i.e. meal feed or pelleted feed. Nowadays, domestic animals and productive livestock, e.g. pigs, also cattle, sheep and poultry, are often kept in animal housing which is equipped with the most modern, fully automatic feeding installations. In these, the feed is offered according to the age and weight of the animal, in quantities adapted to nutrient requirements, fully automatically in the feeding trough.

In such fully automated plants, the much-discussed feed pellets are used. The feed in question is compressed, highly compacted energy dry feed on a vegetable and/or animal basis, which may be enriched with additives such as amino acids, vitamins and minerals. These feed pellets are no more than artificial, free-flowing, round or oblong grains, balls or even rod-shaped objects, depending on the manufacturing process, of a uniform size tailored to the age and weight of the animals, which may be from a few millimetres for poultry to ca. one centimetre for adult pigs and cattle. Feed pellets are prepared by commercial feed mills by grinding the organic starting material, mixing the components in the desired composition and finally compressing into pellets, then they are filled into sacks and delivered to the animal keeper, who pours them into the distribution equipment. An important advantage of these pellets is their simple handling which is a result of their uniformity, their fluidity and their stability in storage. They can be easily filled and dispensed, transported via conveyor belts or pipelines and administered to each animal in a precisely proportioned amount, all fully automatically. In addition, pellets take up substantially less room than fresh feed and are eaten by the animals willingly and without problems.

There is therefore the possibility of adding to these pellets not only amino acids and other vital substances such as vitamins and minerals, but also antibiotics when needed. This is already being carried out in practice, but in the case of valnemulin meets with the particular difficulties as depicted, which are characteristic of this class of substance and will be explained more fully below.

It has been shown that valnemulin is rather unstable when producing pelleted feed, particularly when in contact with the feed material, especially constituents of vegetable or animal origin. This leads to substantial losses already at the preparation stage. In the preparation of pelleted feed, the dried organic starting material of animal or vegetable origin is ground, mixed intimately with the admixtures, vitamins, trace elements and other additives, i.e. substantially homogenized, and then optionally moistened with ca. 5 to 10% by weight water and compressed into feed pellets at elevated temperatures ranging from ca. 60 to 100°C at pressures of ca. 1 to 100 kbar. Short-term, local temperature peaks in the press, so-called flashes, even reach 200°C for a short time. The retention time of the mass in the press is generally from ca. 5 to 180 seconds and depends *inter alia* on the size of the pellets.

Whereas valnemulin in the form of the dried, amorphous hydrochloride salt withstands these temperatures for a short time without significant decomposition and can be stored at room temperature even for a few months without any measurable loss of active ingredient, this active ingredient decomposes relatively rapidly under pressure and in intimate contact with animal or vegetable feed constituents and at the prevailing elevated temperatures. Contact with the constituents of the feed appears precisely to catalyse the decomposition process. Even if the phase involving the raised pressure and elevated temperature is kept as short as is technically possible, and the finished pellets are immediately cooled to room temperature directly after the compression process, one quarter to one third of the active ingredient, namely valnemulin, is still lost. The loss of active ingredient leads without doubt to problems of correct dosaging for the animal and thus to success of the treatment, as well as a considerable increase in costs of the end product.

It has also been shown that intact valnemulin in the pellets is considerably less stable in storage than for example the dry, amorphous hydrochloride. Breakdown of the active ingredient continues in the finished pellets even at room temperature. Even after three months, the content of active ingredient drops to below 60%. This relative instability has also led to the fact that exact dosaging of the active ingredient in the form of feed pellets could only be previously ensured for ca. 3 weeks after production of the pellet. Therefore, the animal keepers were forced to use only relatively freshly produced pellets. They could not pursue meaningful long-term storage and had to place a new production request with the feed mills every four to six weeks, so that fresh feed with a guaranteed content of antibiotics would be available to them. Though technically feasible, there is a high degree of logistics involved and this meant that the feed mills always had to produce small orders which did not necessarily suit their production programme, leading to enormous cleaning efforts between each batch in order to avoid cross-contamination, and thus to additional expense of the pellets.

For these reasons, a number of efforts were made to stabilize valnemulin and other representatives of the class of pleuromutilins, so that they withstand the raised temperatures and pressures during production of the pellets, without loss of active substance, and also have long-term practical stability in the form of the prepared pellets.

These unsuccessful attempts include e.g. (1) a reduction in active ingredient surface area by compressing into grains, with many different grain sizes being tried out, (2) sealing said grains of active ingredient with many different protective layers, for example with gelatins or different sugars and lacquers, (3) enclosing the active ingredient in porous materials, e.g. in various cefluloses, starches, silicic acids or zeoliths with and without protective layers; or (4) chemical modification of the macrocyclic basic framework of the active ingredient. In a few cases, chemical modification did indeed lead to improved stability of the molecule *per* se, but at the same time led to a toss of efficacy.

In EP0524632, by forming a complex with cyclodextrin, an attempt was made to raise stability in storage of dry feed, which was also partially successful.

Another more successful attempt to stabilize pleuromutilins such as the aforenamed valnemulin is described in WO 03/45354. In this, the active ingredient is enclosed in microspherules in a special procedure; these microspherules are then added to the dry animal feed and compressed into pelleted feed at a higher pressure and elevated temperature. However, this procedure is technically very complex and leads to a substantial increase in the cost of the pelleted feed.

Other patent references, e.g. WO 01/41768, describe the production of injection solutions and the thereby associated technical difficulties. In the case of injections, *inter alia* undesired side effects are observed, ranging from mild skin irritation to poorly healing necrosis. This is also one of the reasons that valnemulin has mainly been used orally until now. Users have also sometimes complained that aqueous solutions show no depot action. A further problem is that valnemulin in free form, as the so-called valnemulin base, is also extraordinarily unstable and was therefore produced as the amorphous hydrochloride salt, and since then was preferably used in this form to treat bacterial infections. Injection forms with improved tolerance are described in the above WO 01/41758. Preparations with amorphous valnemulin hydrochloride lead, in isolated cases, to palatability problems if they are offered orally, especially in liquid form.

It is therefore evident that, as before, there is a great need for an application form of valnemulin, which is simple and therefore inexpensive to produce, which thanks to its precise mixability with foodstuffs can be measured out efficiently and reproducibly, and which leads to the desired stability during preparation and storage of dry animal feed.

In a few patent references, for example in EP-0,153,277 or WO 03/082260, salts of valnemulin with organic acids have already been mentioned or even specifically named. For example, in EP-0, 153,277, the hydrogen fumarate, the fumarate and the naphthalene-1,5-sulphonate of valnemulin are named specifically. However, when studying this reference, it is established that these are purely hypothetical substances, since there is no report on the preparation thereof nor on any kind of chemical or biological data. The only salt disclosed in EP-0, 153,277 is amorphous hydrochloride. Consequently, as before, salts of valnemulin with organic acids, especially in crystalline form, are new. Even crystalline hydrochloride is not described anywhere.

Gould P L: "Salt selection for basic drugs", International J. Pharmaceutics 1986, 201-217 and Berge S M et al.:"Pharmaceutical salts", J Pharmaceutical Sciences Vol. 66 (1), 1-19 (1977) describe the role of salts in general on the overall properties of a drug.

We have now surprisingly succeeded in preparing an application form of valnemulin with certain salts of organic acids, which combines the said stability and palatability advantages and can be produced inexpensively, whereby the salts with the organic acids are crystalline, since their stability over the amorphous salts is substantially increased and, in addition, the crystalline salts are far better accepted by animals when administered orally. Palatability of oral forms of administration in animal medicine can be crucial to success or failure of treatment. It is therefore also an aim of the present invention to provide an application form with improved palatability.

The present invention solves this problem in an optimum way by reacting valnemulin with organic acids to form an acid addition salt, which surprisingly has high crystallinity and stability in storage. In principle, all physiologically acceptable organic acids are suitable. Examples of suitable acids are monocarboxylic acids such as formic acid, acetic acid, propionic acid, ascorbic acid, glycolic acid, lactic acid, pyruvic acid or mandelic acid, but also dicarboxylic acids such as oxalic acid, malonic acid, succinic acid, aspartic acid, glutamic acid, glutaric acid, adipic acid, pimelic acid, suberic acid, maleic acid, fumaric acid, phthalic acid, isophthalic acid, terephthalic acid, malic acid or tartaric acid, or even tricarboxylic acids, e.g. citric acid. In general, the enantiomer-pure mono-and dicarboxylic acids are to be preferred. D-tartaric acid and fumaric acid are especially suitable, and in particular D-tartaric acid.

The advantage of the acid addition salts produced according to this invention also lies in the good crystallinity, which leads to high purity and thus safety in using the application forms prepared therefrom.

One general process for producing pure valnemulin salts with organic acids consists e.g. in
a) reacting crude valnemulin hydrochloride with a base to form the free valnemulin base,
b) if required extracting the free base with an organic solvent and isolating by conventional processes,
c) reacting the valnemulin base in an organic solvent or a mixture of organic solvents and optionally water with an organic acid, optionally at elevated temperature, and
d) if required after adding seeding crystals, if required by slowly cooling the reaction solution, allowing the valnemulin acid addition salt to crystallise.

In the case of di- or tri-basic organic acids, equimolar or equinormal amounts of acid may be reacted with the valnemulin base, equimolar amounts being preferred.

Suitable bases for releasing the valnemulin base are e.g. alkali metal or alkaline earth metal hydroxides, hydrides, amides, alkanolates, acetates or carbonates, alkylamines, alkylenediamines, optionally N-alkylated, optionally unsaturated, cycloalkylamines, ammonium hydroxides, as well as carbocyclic amines. Those which may be mentioned by way of example are sodium hydroxide, hydride, amide, methanolate, acetate, carbonate, potassium tert.-butanolate, hydroxide, carbonate, hydride, calcium hydride, triethylamine, diisopropylethylamine, triethylenediamine, cyclohexylamine, N-cyclohexyl-N,N-dimethylamine, benzyltrimethylammonium hydroxide, as well as 1,5-diazabicyclo[5.4.0]undec-5-ene (DBU). Preference is given to alkali metal hydroxides, especially sodium hydroxide.

Suitable solvents for extracting the free valnemulin base are aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; esters, such as methyl acetate, ethyl acetate, isopropyl acetate or butyl acetate; or ethers, such as diethylether, dipropyl ether, diisopropyl ether, dibutyl ether, tert.-butylmethyl ether, ethyleneglycol monomethyl ether, ethyleneglycol monoethyl ether, ethylene glycoldimethyl ether, dimethoxydiethyl ether, tetrahydrofuran or dioxane, or mixtures thereof. Preference is given to ethers, especially tert.-butylmethyl ether.

Suitable solvents for reacting the valnemulin base with an organic acid are aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; esters, such as methyl acetate, ethyl acetate, isopropyl acetate or butyl acetate; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethylether, dimethoxydiethy lether, tetrahydrofuran or dioxane; ketones such as acetone, methyl ethyl ketone or methyl isobutyl ketone; alcohols such as methanol, ethanol, propanol, isopropanol, butanol, ethylene glycol or glycerol; amides such as N,N-dimethylformamide, N,N-diethyl-formamide, N, N-dimethylacetamide, N-methylpyrrolidone or hexamethylphosphoric acid triamide; or nitriles such as acetonitrile or propionitrile; and sulfoxides, such as dimethyl sulfoxide, or mixtures thereof with and without water.

Preference is given to esters and ketones, as well as mixtures thereof with water, especially mixtures of ethyl acetate, acetone and water. The solvent mixtures that are especially preferred are those consisting of 70% by volume to 90% by volume of ethyl acetate, 5% by volume to 25% by volume of acetone and 0 to 5% by volume of water, in particular mixtures consisting of 75% by volume to 80% by volume of ethyl acetate, 20% by volume to 25% by volume of acetone and 1% to 2% by volume of water.

In the following, by way of example, preparation processes are described for valnemulin acid addition salts from the reaction of free valnemulin or its hydrochloride with organic acids. All temperatures are given in degrees Celsius.

### Preparation examples

### Example 1: Preparation of valnemulin-D-hydrogen tartrate by exchange of salts

a) 30.1 g of valnemulin hydrochloride are added whilst stirring to 300 ml of water which has been heated to 30-35°C. Afterwards, 150 ml of tert.-butylmethyl ether are added, and the pH is adjusted to 8 to 9 with Ca. 5 ml of 10 n sodium hydroxide solution. After stirring for minutes at 30-35°C, the organic phase is separated and washed twice, each time with ml of water. The organic solvent is subsequently distilled off under normal pressure.
b) The residue of evaporation, consisting of non-purified valnemulin, is dissolved at Ca. 40°C in a prepared solution of 50 ml of ethanol and 7.5 g of D-tartaric acid, and the mixture stirred with 350 ml of methyl ethyl ketone. Upon reaching a temperature of 35°C, seeding crystals are added whilst stirring thoroughly, whereupon the title compound slowly crystallizes. The suspension is stirred for a further Ca. 8 hours, whilst cooling passively. The product is subsequently filtered, washed with methyl ethyl ketone, and dried at 50°C. The title compound is thus obtained as white crystals having a m.p. of 130°C.

### Example 2: Preparation of valnemulin D-hydrogen tartrate from the free base

23.5 g of valnemulin are dissolved at 65°C in 240 ml of a solvent mixture consisting of 78% ethyl acetate, 20.5% acetone and 1.5% water, then 6.9 g of D-tartaric acid are added, and the mixture is stirred until a clear solution is obtained. Whilst continuing to stir, 25 mg of seeding crystals are added, and ca. 10 minutes thereafter crystallisation commences. The suspension is stirred for a further hour at boiling temperature, and then cooled to room temperature over the course of 2 hours. The precipitated product is filtered and dried in a vacuum over night at 50°C. The title compound is thus obtained as white crystals having a m.p. of 172°C.

### Example 3: Preparation of valnemulin hydrogen fumarate from the free base

59.3 g of valnemulin are dissolved at 40°C in 220 ml of a solvent mixture consisting of 78% ethyl acetate, 20.5% acetone and 1.5% water, then 12.1 g of fumaric acid are added, and the mixture is stirred until a clear solution is obtained. Afterwards, the mixture is cooled to 30°C and 0.5 g of seeding crystals are added whilst stirring. The mixture is subsequently stirred for a further 3 hours at 30°C and then left to cool to room temperature over night. Afterwards, the mixture is stirred for a further 2 hours at 0°C. Finally, the cold suspension is filtered, the residue washed with ethyl acetate and dried over night in a vacuum at 50°C. The title compound is thus obtained as white crystals having a m.p. of 132°C.

### Stability tests

In order to test the stability of the valnemulin acid addition salts in animal feed, the calculated amount of the acid addition salt (corresponding to a target dose of 100 ppm) is added to ca. 4 kg of wheat-based animal feed and mixed in a high-speed laboratory mixer for 60 seconds to form a first premix (PM1). The ca. 4 kg of premix PM1 is then transferred to a horizontal mixer and mixed with 21 kg of the same feed for a further 6 minutes to form a further premix (PM2). The ca. 25 kg of premix PM2 is transferred to a vertical mixer and intimately mixed with a further 175 kg of the same animal feed for 8 minutes, whereupon a homogeneous, treated mixture is obtained, which contains valnemulin concentrations corresponding to the target dose of 100 ppm.

Prior to further processing, samples of ca. 100 g each are taken from the upper, centre and lower part of the medicinal feed mixture, in order to test its homogeneity. In addition, further samples are taken randomly for use in stability tests.

The finished, treated medicinal feed is now ready for pelleting. With this in mind, it is conveyed to a finishing chamber, to which 3 kg of water and saturated steam are added, in order to adjust the pelleting temperature to 75°C to 85°C. The 200 kg of finished medicinal feed is then fed continuously into the pelleting press of the feed mill over the course of ca. 20 mins. Afterwards, the pellets are dried in batches in a continuous stream of air, and cooled.

To examine the homogeneity of the pelleted medicinal feed, several samples of ca. 100 g each are taken at the start, middle and end of the pelleting process. In addition, samples are taken in a random order from each batch, in order to test the stability in storage.

For the following examples, the amounts of active ingredient per 200 kg of animal feed are 20 g of valnemulin hydrochloride, valnemulin D-hydrogen tartrate, or valnemulin hydrogen fumarate, which corresponds to 100 ppm of valnemulin in the feed.

In the following table 1, by way of example, the stabilities of different acid addition salts of valnemulin in the pelleting procedure for the animal feed are compared.

**Table 1: Loss of active substance in the pelleting procedure at 75°C**

| active substance | average loss in % |
|---|---|
| valnemulin hydrochloride | 11.6 |
| valnemulin D-hydrogen tartrate | 0.3 |
| valnemulin hydrogen fumarate | 0.2 |

These data show the extraordinarily high stability of the addition salts of valnemulin with organic acids, compared with the known hydrochloride.

## Claims

1. A salt consisting of valnemulin of formula I 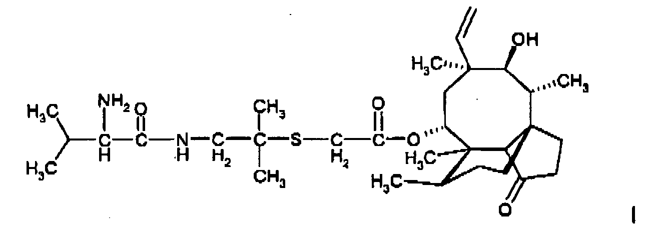 and an organic acid, **characterized in that** said salt is present in crystalline form.

2. A salt according to claim 1, **characterized in that** the organic acids are selected from the group consisting of monocarboxylic acids, dicarboxylic acids and tricarboxylic acids.

3. A salt according to claim 1, **characterized in that** the organic acid is selected from the group consisting of formic acid, acetic acid, propionic acid, ascorbic acid, glycolic acid, lactic acid, pyruvic acid, mandelic acid, oxalic acid, malonic acid, succinic acid, aspartic acid, glutamic acid, glutaric acid, adipic acid, pimelic acid, suberic acid, maleic acid, fumaric acid, phthalic acid, isophthalic acid, terephthalic acid, malic acid, tartaric acid, and citric acid.

4. A salt according to claim 1, **characterised in that** the organic acid is selected from the group consisting of an enantiomer-pure mono-and dicarboxylic acid.

5. A salt according to claim 1, **characterized in that** the organic acid is selected from the group consisting of D-tartaric acid and fumaric acid.

6. A salt according to claim 1, **characterized in that** the organic acid is D-tartaric acid.

7. Process for the production of a salt according to one of claims 1 to 6, **characterized by**
a) reacting crude valnemulin hydrochloride with a base to form the free valnemulin base,
b) if required extracting the free base with an organic solvent and isolating by conventional processes,
c) reacting the valnemulin base in an organic solvent or a mixture of organic solvents and optionally water with an organic acid, and
d) if required after adding seeding crystals, if required by slowly cooling the reaction solution, allowing the valnemulin acid addition salt to crystallize.

8. Process according to claim 7, **characterized in that** extraction of the valnemulin base takes place in an ether.

9. Process according to claim 7, **characterized in that** extraction of the valnemulin base takes place in tert.-butyl methyl ether.

10. Process according to claim 7, **characterized in that** the reaction of the valnemulin base with an organic acid takes place in a solvent from the group of alcohols and ketones.

11. Process according to claim 10, **characterized in that** the solvent is ethanol and methyl ethyl ketone.

12. Process according to claim 7, **characterized in that** the reaction of the valnemulin base with an organic acid takes place in a solvent from the group of esters and the group of ketones and water.

13. Process according to claim 12, **characterized in that** the solvent mixture consists of ethyl acetate, acetone and water.

14. Process according to claim 12, **characterized in that** the solvent mixture consists of 70% by volume to 90% by volume of ethyl acetate, 5% by volume to 25% by volume of acetone and 0 to 5% by volume of water.

15. Process according to claim 12, **characterized in that** the solvent mixture consists of 75% by volume to 80% by volume of ethyl acetate, 20% by volume to 25% by volume of acetone and 1 to 2% by volume of water.

16. Use of a salt according to one of claims 1 to 6 for producing a medicament for the treatment of bacterial infections in warm-blooded animals.

17. Use of a salt according to one of claims 1 to 6 as an animal feed additive.

18. Animal feed pellets, **characterized in that** they contain an active amount of at least one salt according to one of claims 1 to 6.

19. Use of a salt according to one of claims 1 to 6 as a drinking water additive.

## Patentansprüche

1. Salz, bestehend aus Valnemulin der Formel I 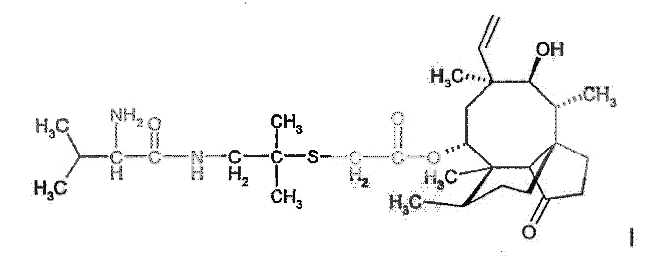 und eine organische Säure, **dadurch gekennzeichnet**, das Salz in kristalliner Form vorliegt.

2. Salz nach Anspruch 1, **dadurch gekennzeichnet**, die organischen Säuren ausgewählt sind aus der Gruppe bestehend aus Monocarbonsäuren, Dicarbonsäuren und Tricarbonsäuren.

3. Salz nach Anspruch 1, **dadurch gekennzeichnet, dass** die organische Säure ausgewählt ist aus der Gruppe bestehend aus Ameisensäure, Essigsäure, Propionsäure, Ascorbinsäure, Glykolsäure, Milchsäure, Brenztraubensäure, Mandelsäure, Oxalsäure, Malonsäure, Bernsteinsäure, Asparaginsäure, Glutaminsäure, Glutarsäure, Adipinsäure, Pimelinsäure, Suberinsäure, Maleinsäure, Fumarsäure, Phthalsäure, Isophthalsäure, Terephthalsäure, Äpfelsäure, Weinsäure und Zitronensäure.

4. Salz nach Anspruch 1, **dadurch gekennzeichnet, dass** die organische Säure ausgewählt ist aus der Gruppe bestehend aus einer Enantiomerreinen Mono- und Dicarbonsäure.

5. Salz nach Anspruch 1, **dadurch gekennzeichnet, dass** die organische Säure ausgewählt ist aus der Gruppe bestehend aus D-Weinsäure und Fumarsäure.

6. Salz nach Anspruch 1, **dadurch gekennzeichnet, dass** die organische Säure D-Weinsäure ist.

7. Verfahren zur Herstellung eines Salzes nach einem der Ansprüche 1 bis 6, **gekennzeichnet durch**
a) Umsetzen von rohem Valnemulin-Hydrochlorid mit einer Base, um die freie Valnemulin-Base zu bilden,
b) wenn benötigt, Extrahieren der freien Base mit einem organischen Lösungsmittel und Isolieren **durch** konventionelle Verfahren,
c) Umsetzen der Valnemulin-Base in einem organischen Lösungsmittel oder einer Mischung organischer und gegebenenfalls Wasser mit einer organischen Säure und
d) wenn benötigt, nach Zugabe von Impfkristallen, wenn benötigt **durch** langsames Abkühlen der Reaktionslösung, dem Valnemulin-Säureadditionssalz ermöglichen, zu kristallisieren,

8. Verfahren nach anspruch 7, **dadurch gekennzeichnet, dass** die Extraktion der Valnemulin-Base in einem Ether stattfindet,

9. Verfahren nach Anspruch 7, **dadurch gekennzeichnet, dass** die Extraktion der Valnemulin-Base in tert.-Butylmethylether stattfindet.

10. Verfahren nach Anspruch 7, **dadurch gekennzeichnet, dass** die Reaktion der Valnemulin-Base mit einer organischen Säure in einem Lösungsmittel aus der Gruppe der Alkohole und Ketone stattfindet.

11. Verfahren nach Anspruch 10, **dadurch gekennzeichnet, dass** das Lösungsmittel Ethanol und ist.

12. Verfahren nach Anspruch 7, **dadurch gekennzeichnet, dass** die Reaktion der Valnemulin-Base mit einer organischen Säure in einem Lösungsmittel aus der Gruppe der Ester und der Gruppe der Ketone und Wasser stattfindet.

13. Verfahren nach Anspruch 12, **dadurch gekennzeichnet**, die Lösungsmittel-mischung aus Ethylacetat, Aceton und Wasser besteht.

14. Verfahren nach Anspruch 12, **dadurch gekennzeichnet, dass** die Lösungsmittelmischung aus 70 Volumen-% bis 90 Volumen-% Ethylacetat, 5 Volumen-% bis 25 Volumen-% Aceton und 0 bis 5 volumen-% Wasser besteht.

15. Verfahren nach Anspruch 12, **dadurch gekennzeichnet, dass** die Lösungsmittelmischung aus 75 Volumen-% bis 80 Volumen-% Ethylacetat, 20 Volumen-% bis 25 Volumen-% Aceton und 1 bis 2 Volumen-% Wasser besteht.

16. Verwendung eines Salzes nach einem der Ansprüche 1 bits 6 zur Herstellung eines Medikaments zur Behandlung bakterieller Infektionen in warmblütigen Tieren.

17. Verwendung eines Salzes nach einem der Ansprüche 1 bis 6 als ein Tierfutter-Zusatz.

18. Tierfutter-Pellets, **dadurch gekennzeichnet, dass** sie eine wirksame Menge mindestens eines Salzes nach einem der Ansprüche 1 bis 6 anhalten.

19. Verwendung eines Salzes nach einem der Ansprüche 1 bits 6 als ein Trinkwasser-Zusatz.

## Revendications

1. Sel composé de valnémuline de formule I 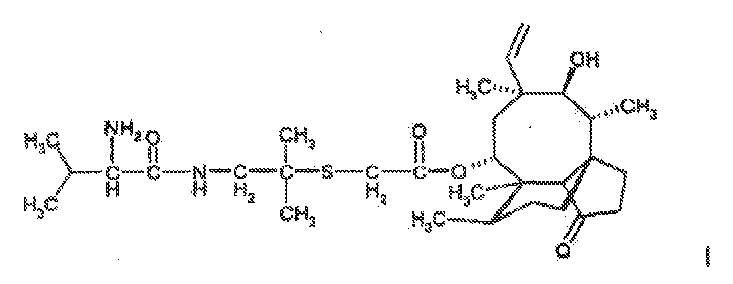 et d'un acide organique, **caractérisé en ce que** ledit sel est présent sous forme cristalline,

2. Sel selon la revendication 1, **caractérisé en ce que** les acides organiques sont sélectionnés à partir du groupe composé d'acides monocarboxyliques, d'acides dicarboxyliques et d'acides tricarboxyliques.

3. Sel selon la revendication 1, **caractérisé en ce que** l'acide organique est sélectionné à partir du groupe composé d'acide formique, d'acide acétique, d'acide propionique, d'acide ascorbique, d'acide glycolique, d'acide lactique, d'acide pyruvique, d'acide mandélique, d'acide oxalique, d'acide malonique, d'acide succinique, d'acide aspartique, d'acide glutamique, d'acide glutarique, d'acide adipique, d'acide pimélique, d'acide subérique, d'acide maléique, d'acide fumarique, d'acide phtalique, d'acide isophtalique, d'acide téréphtalique, d'acide malique, d'acide tartrique et d'acide citrique.

4. Sel selon la revendication 1, **caractérisé en ce que** l'acide organique est sélectionné à partir du groupe composé d'un acide mono- et dicarboxylique énantiomère-pur.

5. Sel selon la revendication 1, **caractérisé en ce que** l'acide organique est sélectionné à partir du groupe composé d'acide D-tartrique et d'acide fumarique.

6. Sel selon la revendication 1, **caractérisé en ce que** l'acide organique est l'acide D-tartrique.

7. Procédé de production d'un sel selon l'une quelconque des revendications 1 à 6, **caractérisé**
a) **par** la réaction du chlorhydrate de valnémuline brut avec une base de façon à former la base libre de valnémuline,
b) si nécessaire, par l'extraction de la base libre avec un solvant organique et par l'isolation à l'aide de procédés conventionnels,
c) par la réaction de la base de valnémuline dans an solvant organique ou un mélange de solvants organiques et, éventuellement, de l'eau avec un acide organique, et
d) si nécessaire, suite à l'ajout de petits cristaux, si nécessaire en refroidissant lentement la solution de réaction, en laissant le sel d'addition d'acide se cristalliser.

8. Procédé selon la revendication 7, **caractérisé en ce que** l'extraction de la base de valnémuline a lieu dans de l'éther.

9. Procédé selon la revendication 7, **caractérisé en ce que** l'extraction de la base de valnémuline a lieu dans du tert-butyl méthyl éther.

10. Procédé selon la revendication 7, **caractérisé en ce que** la réaction de la base de valnémuline avec un acide organique a lieu dans un solvant provenant du groupe composé d'alcools et de cétones.

11. Procédé selon la revendication 10, **caractérisé en ce que** le solvant est de l'éthanol et du méthyl éthyl cétone.

12. Procédé selon la revendication 7, **caractérisé en ce que** la réaction de la base de valnémuline avec un acide organique a lieu dans un solvant provenant du groupe composé d'esters et du groupe composé de cétones et d'eau.

13. Procédé selon la revendication 12, **caractérisé en ce que** le mélange de solvants se compose d'éthyl acétate, d'acétone et d'eau.

14. Procédé selon la revendication 12, **caractérisé en ce que** le mélange de solvants se compose de 70%, par volume à 90% par volume d'éthyl acétate, de 5% par volume à 25% par volume d'acétone et de 0% à 5% par volume d'eau.

15. Procédé selon la revendication 12, **caractérisé en ce que** le mélange de solvants se compose de 75% par volume à 80% par volume d'éthyl acétate, de 20% par volume à 25% par volume d'acétone et de 1% à 2% par volume d'eau.

16. Utilisatlon d'un sel selon l'une quelconque des revendications 1 à 6 dans la production d'un médicament destiné au traitement d'infections bactériennes chez les animaux à sang chaud.

17. Utilisation d'un sel selon l'une quelconque des revendications 1 à 6 sous forme d'un additif alimentaire pour animaux.

18. Pastilles alimentaires pour animaux, **caractérisées en ce qu'**elles contiennent une quantité active d'au moins un sel selon l'une quelconque des revendications 1 à 6.

19. Utilisation d'un sel selon l'une quelconque des revendications 1 à 6 en tant qu'additif à l'eau de boisson.
